# EUROPEAN PATENT APPLICATION

(11) **EP 2 236 484 A1**
(43) Date of publication of application: **06.10.2010**
(21) Application number: 08794018.5
(22) Date of filing: 09.06.2008
(51) Int. Cl.: C07C 11/18

(54) **PLANT FOR THE LIQUID PHASE SYNTHESIS OF ISOPRENE FROM ISOBUTYLENE AND FORMALDEHYDE**

(30) Priority: 21.12.2007 RU 2007147962
(71) Applicant: Obshestvo O Ogranichennoi Otvetstvennoctiu "Eurochim SPB-Treiding", St.Petersburg 190020 (RU)
(72) Inventor: BUSYGIN, Vladimir Mikhailovich, Nizhnekamsk 423550 (RU); DYKMAN, Arkadiy Samuilovich, St.Petersburg 194356 (RU); GILMANOV, Khamit Khamisovich, Nizhnekamsk 423570 (RU); POLYAKOV, Sergey Aleksandrovich, St.Petersburg 192177 (RU); FLEGONTOV, Aleksey Michaylovich, St.Petersburg 193318 (RU); FEDORCOVA, Elena Vladimirovna, St.Petersburg 195427 (RU)
(74) Representative: Henrion, Oliver
(86) International application number: PCT/RU2008/000367
(87) International publication number: WO 2009/082260

(57) **Abstract**

The invention relates to a plant for the synthesis of isoprene in a liquid phase from isobutylene and formaldehyde or from the source substances thereof. The inventive plant consists of a dimethyl dioxane synthesis unit detached from two parallel operating trimethyl carbinol synthesis units, and the trimethyl carbinol and dimethyl dioxane produced therein are simultaneously supplied for common decomposition to an isoprene synthesis unit.

Light by-products extracted in a unit for rectifying high-boiling by-products, which unit is located downstream of the dimethyl dioxane synthesis unit, are supplied either to sales or to decomposition together with by-products (methyl-dihydropyrane fraction) which are produced during end-products separation and isoprene monomer extraction, or to sales and to a decomposition unit for decomposition together with the above-mentioned by-products (methyl-dihydropyrane fraction), afterwards to the end-product separation and isoprene monomer extraction unit.

The end-product separation and isoprene monomer extraction unit is directly connected to the soprene synthesis unit, the decomposition unit and to the dimethyl dioxane synthesis unit. Said plant makes it possible to reduce the accumulation of high-boiling by-products, to decrease the production costs and to improve the engineering-and-economical performances of the process.

## Description

The present useful model relates to devices, using in the area of production of monomers for synthetic rubber, in particular, it concerns the location and interrelation of vehicles for carrying out of synthesis of isoprene in liquid phase from isobutylene and formaldehyde or substances, which are the sources of isobutylene and formaldehyde, for example, trimethyl carbinol (TMC) or dimethyl dioxane (DMD), in the presence of water solution of acid-catalyst.

The construction of reactor block, which includes two series-connected vehicles - airlift column and shell-and-tube column with external circulation loop, connecting the upper separating and lower reaction zones, with dispensers, located in the lower part of the columns, and equipped by annular narrowings, installed on the pipeline inner surface of the reactor at the uniform height, meant for increasing of actual time of being of reagents in reaction volume, is known (patent of RF 2096076, published in 1994).

This construction partially removes general deficiency of tubular reactors - sharp change of the ratio of gas and liquid phase over the height of the pipe.

The reached effect is based on that, that at the segments of the pipe, located higher, than narrowing, density of moving mixture grows up slightly. However, installation of annular narrowings can't remove other defects of this construction, such as significant steel intensity, complexity of process control, unavoidable losses of isoprene in consequence of inefficiency of output of it from the reaction medium, etc.

The construction of installation for liquid-phase synthesis of isoprene in one stage, consisting of vertically installed hollow reactor, connected in the single block with shell-and-tube heat exchanger, installed in parallel or in alignment, meant for heating of reaction mass and processing of the part of raw materials, is known. The steam is supplied in the tubular space of the heat exchanger, and the reaction mass circulates through the pipes. At the bottom of cylindrical section of the reactor in the line with the axis of the pipe, connecting the reactor with the top part of the heat exchanger, the dispenser is installed, meant for input of the main part of raw materials. The dispenser, equivalent in construction, is installed at the bottom of the heat exchanger (under the tube sheet) in the line with the axis of the pipe, connecting this vehicle with the reactor (over the upper tube sheet). The second pipe goes from the lowest point of the spherical part of the reactor, connecting the reactor with the lower zone of the heat exchanger, located lower, than the tube sheet, and from the highest point of the reactor goes the pipe, meant for the off-set of contact gas to the separator (patent of RF Nº 32706, published in 2003).

This construction provides adequate mixing of the reaction mass and, not least importantly, the effective off-set of isoprene in the current of isobutylene from the reaction zone, however, the critical weakness of it is the presence of external heat exchanger in its composition, resulting in increasing of steel intensity of the installation (expenditures, connected with production of heavy flanges and body of shell-and-tube heat exchanger), and the main weakness is connected with unavoidable heat losses.

Because of the presence of the last mentioned, it is necessary to pre-heat raw materials up to the reaction temperature or overheat them 5-7°C higher than this temperature.

As raw materials are supplied to the reactor with the water solution of the acid-catalyst, this operation unavoidably leads to the losses of raw materials for shaping of additional quantities of co-products and creates conditions for gum formation.

The simplified construction of installation of liquid-phase synthesis of isoprene from isobutylene and formaldehyde, allowing to carry out synthesis in the single reaction zone (in one stage), consisting of the hollow reactor, vertically installed, in which the shell-and-tube heat exchanger is built-in, meant for heat supply, which is necessary for the flow of the reaction, and one or two circulation pipes, connecting the upper and the lower parts of the reactor, the diameter of which is not less, than in five times exceeds the diameter of the pipes of the heat exchanger, is known. The body of the hollow reactor is, in the meantime, the shell of the heat exchanger. The ratio of volumes of the upper and lower parts of the hollow reactor, separated by the built-in heat exchanger, composes the value, which is not less, than 2-2.5. The circulation of the reaction mass is carried out in the pipes of the heat exchanger at the expense of density differences of gas and liquid. For intensification of mixing, the force-feed pump circulation may be used. Both parts of the reactor contain the dispensers, meant for supplying of raw materials.

The dispensers represent the system of pipes with holes with the diameter of 2-5 mm, installed in parallel at the central (feeding) pipe. The square of the dispensers, calculated over their overall sizes, composes at most 25% of cross-section of the containers, in which they are installed (Patent of RF Nº 42185, published in 2004).

The advantages of the described installation include the reduction in metal consumption of the reactor block at the expense of simplifying of the construction **-** excluding of flange connections and using of the body of the reactor as the shell of the heat exchanger. However, during the short work period of the installation, additional quantity of co-products appears, that increases gum formation and leads to losses of raw materials.

The installation of synthesis of isoprene from isobutylene and formaldehyde in liquid phase in the presence of homogeneous acid catalyst, which doesn't provide separation of synthesis intermediates, is known (Patent of USA Nº 4511751, 1985, patent of USA Nº 4593145, 1986).

The installation includes 2-4 series-connected reactors of interaction of acid water solution of formaldehyde with isobutylene and/or trimethyl carbinol at the temperature of 150-220°C and pressure, in 1-2,5 times exceeding the vapor pressure of the reaction mixture at these temperatures. Isobutylene and/or trimethyl carbinol are supplied only to the first reactor, and formalin - to each reactor. Isoprene, water, unreacted raw products are run off from each reaction zone and are entered to the following one with the stripping of them from the last reaction zone. The yield of isoprene for transformed formaldehyde and isobutylene is not enough and composes 52 and 74 %, respectively.

The closest analogue of the offered installation of liquid-phase synthesis of isoprene from isobutylene and formaldehyde is the installation, described in the patent of RF Nº 2280022, published on 20.07.2006.

The installation consists of several connected reactors (blocks) of synthesis of precursors of isoprene and the block of dissolution of resulting products. Between the block of synthesis and block of dissolution, one or two rectification columns are installed. The installation includes the block of product separation, forming in the reactor of dissolution.

The disadvantage of the work of the described installation is appearing of a great quantity of higher boiler co-products and their concentration in the system, resulting in formation of antifloating emulsions and hard deposits on the work equipment and its plugging, especially during operation of the installation in industrial conditions. It is conditional upon the ingress of higher boiler co-products (HCP), especially, their heavy part - formals of dioxane alcohols, appearing during synthesis of precursors of isoprene, in particular, DMD, into the block of dissolution, which is working in the conditions of high temperatures of exploitation (130-170°C) and great concentration of acid catalyst. In this case, HCP, which are the mixture of dioxane alcohols and their derivatives, turn into so-called «green oil», which may be used only as low-calorie fuel, while HCP became common use in the national economy and their price is much higher, than the cost of raw materials.

The transformation of HCP in such a co-product as «green oil» sharply increases the prime cost of the process. Besides, the light part of HCP may be dissolved jointly with the fraction of pyrans into feedstock and isoprene.

In the work of installation over the closest analogue, the heterogeneous-catalytic dissolution of the pyran fraction is not provided, that also worsens technical and economic parameters of the process.

Using in the work of the installation, described in the closest analogue, as the source of isobutylene of the mixture of TMC and isobutylene in the block of synthesis of isoprene also reduces the selectiveness of the process and reduces its expendable parameters.

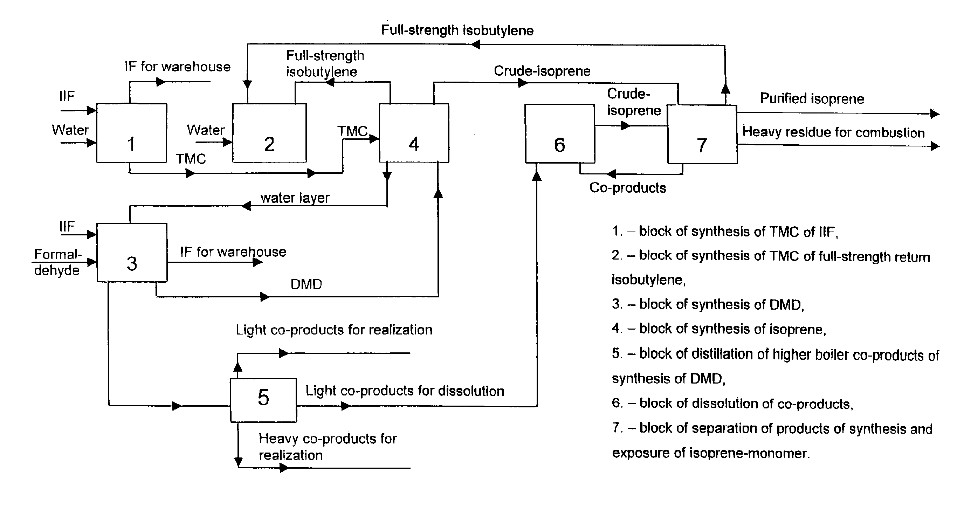

With the purpose of reducing of the prime cost of isoprene and improvement of technical and economic parameters of the process of synthesis of isoprene from isobutylene and formaldehyde, the installation is offered, consisting of the blocks, shown on the enclosed figure, where:
1. - block of synthesis of TMC of isobutane-isobutylene fraction (IIF),
2. - block of synthesis of TMC of full-strength return isobutylene,
3. - block of synthesis of DMD,
4. - block of synthesis of isoprene,
5. - block of distillation of higher boiler co-products of synthesis of DMD,
6. - block of dissolution of co-products,
7. - block of separation of products of synthesis and exposure of isoprene-monomer.

The installation is working in the following way.

For obtaining of the necessary quantity of TMC, IIF and water are supplied to the block 1. Synthesis of TMC is conducting in the multiple-shell reactors, filled by the cation exchanger. The return fraction of IIF from the block is delivered to the warehouse.

The formed TMC, after the separation from the return IIF, is supplied to the block 4 of synthesis of isoprene, where, in the meantime, DMD is supplied from the block 3. The necessary quantity of DMD is synthesized in the block 3, and IIF, formaldehyde and water layer from the block 4 are supplied there for this purpose. The return fraction of IIF from the block 3 is delivered to the warehouse.

The appearing in the block 4 isoprene-containing fraction is supplied for separation of the products of synthesis and exposure of isoprene-monomer in the block 7, and the full-strength isobutylene, driven away in the block 4 and in the block 7, returns to the block 2 of synthesis of TMC of the full-strength isobutylene. In the meantime, water is supplied for synthesis to the block 2.

The higher boiler co-products, appearing during synthesis of DMD, from the block 3 are supplied to the block 5 of rectification of higher boiler co-products of synthesis of DMD, in which the division in light co-products and heavy co-products takes place, which next are delivered for realization. All or the part of the light higher boiler products may be delivered to the block 6 of dissolution of co-products.

In the meantime, co-products are supplied to the block 6 (fraction of 4-methyl-5,6-dihydro-α-pyran (MDHP)) from the block 7, appearing during synthesis of isoprene. The isoprene-containing fraction from the block 6 is delivered for dissolution of products of synthesis and exposure of isoprene-monomer to the block 7, in which the exposure of the purified isoprene and separation of co-products take place.

The useful model contains technical solutions, allowing to reduce specific metal content of the process, increase productivity, to provide continuous work of industrial equipment of the processing installation during one year, and also to use the resulting co-products competently.

Conducting of the process on the basis of TMC, without applying of pure isobutylene in the block 4, allows to perform the process at smaller proportions of reagents (source of isobutylene / source of formaldehyde), due to the fact, that dehydration of TMC is carried in the volume of water phase, in which formaldehyde and acid-catalyst are located, by virtue of that, the probability of «intercept» of isobutylene by formaldehyde sharply increases and, respectively, the speed of their interaction increases.

In the time of industrial use of the offered installation, the following parameters are achieved:
Using of the block 6 of dissolution of co-products, (in particular, 1 t of technical pyran fraction) allows to obtain extra ∼490 kg of isoprene and ∼220 of formaldehyde, that significantly (for ∼5%) reduces consumption indexes of raw materials.

The realization of heavy fraction of HCP on the basis of ∼140 kg for 1t of isoprene, obtained in the block 5, allows to reduce the prime cost of the target product ∼ for 5%.

## Claims

1. An installation for liquid-phase synthesis of isoprene from isobutylene and formaldehyde, comprising a unit for synthesis of precursor-products of isoprene (trimethyl carbinol, dimethyl dioxane), a unit for decomposition of products, being formed in the synthesis, a unit for isolation and purification of the desired product, **characterized in that**, that it consists of a dimethyl dioxane synthesis unit detached from two trimethyl carbinol synthesis units working in parallel (a unit of synthesis from isobutane-isobutylene fraction and a unit of synthesis from recycled concentrated isobutylene, isolated from an isoprene synthesis unit as well as from a unit for separation of synthesis products and isolation of isoprene-monomer), wherein trimethyl carbinol and dimethyl dioxane formed in said units are simultaneously supplied to the isoprene synthesis unit for joint decomposition, and wherein downstream of the dimethyl dioxane synthesis unit a unit for rectification of high-boiling co-products formed in said dimethyl dioxane synthesis unit is installed, from which separated light co-products are sent to realization or to decomposition jointly with co-products (methyldihydropyran fraction) obtained in the separation of synthesis products and isolation of isoprene-monomer or are sent to realization and to decomposition jointly with co-products (methyldihydropyran fraction) obtained in the separation of synthesis products and isolation of isoprene-monomer, said light co-products entering the unit for decomposition; and wherein the unit for separation of synthesis products and isolation of isoprene-monomer is connected directly to the isoprene synthesis unit, to the unit for decomposition of co-products and to the dimethyl dioxane synthesis unit.
